(19) Europäisches Patentamt
European Patent Office
Office européen des brevets

(11) EP 0 569 276 B1

(12) **FASCICULE DE BREVET EUROPEEN**

(45) Date de publication et mention de la délivrance du brevet:
**29.01.1997 Bulletin 1997/05**

(51) Int Cl.[6]: **C07C 215/28**, A61K 31/135

(21) Numéro de dépôt: **93401135.4**

(22) Date de dépôt: **03.05.1993**

(54) **Nouvelle application des enantiomères dérivés du (S)-amino-2-dichloro-3,4-benzyl)-2-propanol-1**

Neue Verwendung von (S) Amino-2-dichloro-3,4-benzyl)-2-propanol-1 Enantiomere Derivate

Novel use of enantiomers derived from (S)-2-amino-3-(3,4-dichlorobenzyl)-1-propanol

(84) Etats contractants désignés:
**AT BE CH DE DK ES FR GB GR IE IT LI LU MC NL PT SE**

(30) Priorité: **05.05.1992 FR 9205519**

(43) Date de publication de la demande:
**10.11.1993 Bulletin 1993/45**

(73) Titulaire: **INSTITUT DE RECHERCHE JOUVEINAL (I.R.J)**
**94265 Fresnes Cedex (FR)**

(72) Inventeurs:
- **Aubard, Gilbert**
  **F-91120 Palaiseau (FR)**
- **Bure, Jacques**
  **F-92200 Neuilly-sur-Seine (FR)**
- **Calvet, Alain**
  **F-94240 L'Hay-Les-Roses (FR)**
- **Gouret, Claude-Jean**
  **F-92190 Meudon (FR)**
- **Grouhel, Agnès**
  **F-92190 Meudon (FR)**
- **Junien, Jean-Louis**
  **F-92310 Sevres (FR)**

(56) Documents cités:
**EP-A- 0 237 366**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 569 276 B1

## Description

La présente invention a pour objet une nouvelle utilisation des dérivés du (S) -amino-2- (dichloro-3,4-benzyl) -2-propanol-1, pour l'obtention de médicaments destinés au traitement d'états allergiques.

Au brevet européen publié sous le N° 0 237 366, il est décrit des amino alcools de formule :

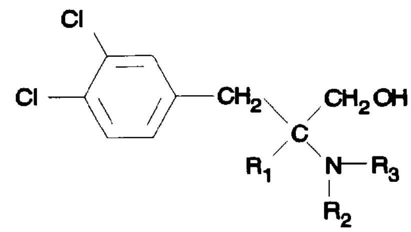

dans laquelle :

$R_1$ est alkyle inférieur,
$R_2$ est H ou alkyle inférieur,
$R_3$ est H, alkyle inférieur, alkényle inférieur, phénylalkyle inférieur ou cycloalkylalkyle inférieur ayant de 3 à 6 atomes de carbone dans le cycle, ou, $R_2$ et $R_3$ forment ensemble, avec l'atome d'azote auquel ils sont reliés, un hétérocycle saturé de cinq à sept chaînons pouvant comprendre, comme second hétéroatome non relié directement à l'atome d'azote, un oxygène, un soufre ou un azote, ce dernier hétéroatome d'azote pouvant porter un substituant alkyle en $C_1$ à $C_4$, les formes racémiques ou optiquement actives de ces amino alcools et leurs sels d'addition avec des acides.

Les produits de cette invention montrent à la fois des propriétés psychotropes et analgésiques particulièrement appropriées aux traitements des troubles psychopathologiques et neuropathologiques, ainsi qu'aux syndromes douloureux d'étiologies diverses.

Or, on a maintenant trouvé que, dans l'ensemble des composés particuliers couverts par la formule générale de l'invention européenne N° 0 237 366, des énantiomères de configuration (S) présentent des propriétés antihistaminiques et antiallergiques inattendues qui sont particulièrement appropriées aux traitements de symptomatologies provoquées par des allergènes divers et en particulier par une libération d'histamine.

La présente invention concerne, au titre d'application nouvelle, l'utilisation des énantiomères de configuration (S), selon la règle établie par Cahn-Ingold-Prelog, qui répondent à la formule (I)

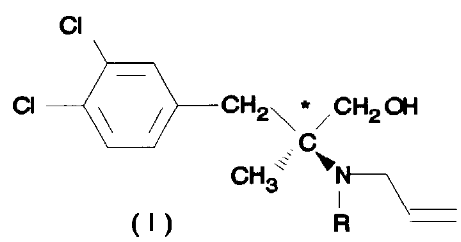
(I)

dans laquelle R est l'hydrogène ou un radical méthyle, leurs sels d'addition avec les acides organiques ou minéraux non toxiques, à la préparation de médicament antiallergique notamment antihistaminique.

Les sels d'addition sont ceux obtenus avec des acides thérapeutiquement acceptables parmi lesquels, on cite à titre d'exemples les acides acétique, benzènesulfonique, camphosulfonique, citrique, éthanesulfonique, fumarique, bromhydrique, chlorhydrique, lactique, maléique, malique, méthanesulfonique, nitrique, pamoïque, phosphorique, salicylique, stéarique, succinique, sulfurique, tartrique.

La préparation des sels est réalisée par des méthodes habituelles à l'homme de l'art, comme la réaction d'addition de la base du produit (I) sur un des acides, en solution dans un solvant miscible à l'eau, comme l'éthanol ou l'acétone, ou non miscible comme le chloroforme ou le dichlorométhane, puis à la séparation du sel formé par concentration et/ou refroidissement du milieu réactionnel ou encore par précipitation du sel par un solvant dans lequel il est insoluble comme par exemple l'éther diéthylique.

Le procédé de préparation des composés (I) consiste soit à dédoubler leurs correspondants racémiques qui sont

décrits au brevet européen N° 0 237 366, soit à en réaliser la synthèse chimique à partir d'énantiomères précurseurs de configuration (S).

Le procédé mettant en oeuvre le dédoublement des composés racémiques consiste à utiliser un acide optiquement actif pour obtenir avec le composé racémique traité soit deux sels d'addition diastéréoisomères soit deux esters optiquement actifs que l'on sépare et à partir desquels on génère par un traitement approprié l'énantiomère de configuration (S) qui est encore nommé dans le texte "eutomère", et par ailleurs, l'antipode de configuration (R) qui est considéré comme inactif et nommé dans le texte "distomère". On peut encore utiliser une méthode de résolution directe du composé racémique par chromatographie liquide de haute performance (CLHP). Plus précisément ces préparations consistent :

a) En ce qui concerne le procédé de résolution au moyen de sels diastéréoisomères, il consiste à faire réagir dans un solvant approprié le composé racémique comprenant le mélange à parties égales d'eutomère et de distomère avec l'acide optiquement actif pour former deux sels diastéréoisomères qui sont séparés par leur différence de solubilité dans le solvant utilisé. Dans ces conditions, le diastéréoisomère de plus faible solubilité précipite, il est isolé par filtration.

Les sels obtenus séparés peuvent être recristallisés dans des solvants sélectifs pour obtenir une pureté optique satisfaisante ou bien être traités, généralement en milieu basique, pour régénérer les amino alcools énantiomères respectifs. Ceux-ci peuvent être alors purifiés par recristallisation ou encore être engagés dans une seconde étape de purification par une nouvelle formation de sels diastéréoisomères et ce, avec éventuellement un acide optiquement actif différent de celui utilisé précédemment.

Les énantiomères d'acides couramment utilisés pour la préparation de sels diastéréoisomères selon ce procédé sont pour exemples ceux de l'α-phénylglycine, α-phénylalanine et leurs dérivés N-carboxylés, de l'acide malique, l'acide mandélique, l'acide tartrique et leurs dérivés d'estérification par des acides, ou encore l'acide camphanique, l'acide 3-bromocampho-8-sulfonique et ses isomères de position, l'acide α-méthoxy-α-trifluorométhylphénylacétique.

Les énantiomères de ces acides sont accessibles dans le commerce et d'un emploi bien connu à l'homme de l'art, ce qui rend leur utilisation appropriée à la résolution des racémiques comprenant les eutomères de formule (I) par préparation puis séparation de sels diastéréoisomères ou pour la préparation de diastéréoisomères pour purification d'un produit préalablement enrichi en un eutomère (I).

La préparation des sels diastéréoisomères consiste à faire réagir pour une mole d'amino alcool à séparer ou purifier, de 0,25 à 2,00 moles de l'énantiomère d'acide en solution dans un solvant ou un mélange de solvants que l'on préfère miscibles totalement ou partiellement avec l'eau, et que l'on choisit habituellement parmi les alcools, les cétones, les éthers de faible poids moléculaire ou encore l'acétonitrile.

De préférence, on effectue la réaction en solution dans l'éthanol, le méthanol ou l'acétone en faisant réagir pour une mole de produit à traiter de 0,5 à 1,25 moles de l'énantiomère d'acide, à une température comprise entre 20° C et celle de l'ébullition du solvant employé ce qui est généralement préféré. La salification est complète après une durée comprise entre 5 minutes et 3 heures. Après quoi le mélange réactionnel est abandonné, d'abord à la température ambiante, puis éventuellement vers 0° C pour cristalliser le diastéréoisomère le moins soluble, cristallisation qui peut être favorisée par ensemencement avec quelques cristaux du sel attendu, s'il a été déjà préparé. A l'achèvement de la cristallisation ou au moment jugé opportun pour obtenir la pureté optique souhaitée, le diastéréoisomère cristallisé est séparé par filtration. Les deux phases contenant chacune un diastéréoisomère plus ou moins purifié sont traitées séparément, soit pour purification du diastéréoisomère, soit pour libération de l'amino alcool énantiomère de son sel ce qui est réalisé par un traitement alcalin en milieu aqueux puis filtration de l'énantiomère libéré ou encore extraction par un solvant organique non miscible à l'eau.

b) Alternativement, le dédoublement du composé racémique ou du mélange comprenant l'eutomère (I) consiste en une estérification par un acide carboxylique optiquement actif pour obtenir un mélange d'esters diastéréoisomères que l'on sépare, puis que l'on saponifie pour obtenir les énantiomères séparés de l'amino alcool dont l'eutomère (I) et le distomère antipode correspondant.

Selon ce procédé, le composé racémique ou partiellement enrichi est engagé dans une réaction d'estérification avec un acide carboxylique optiquement pur ou un de ses dérivés tel qu'un halogénure, un anhydride ou un ester. On utilise par exemple les énantiomères (+) et (-) de l'acide α-méthoxy-α-trifluorométhylphénylacétique et leurs dérivés. La réaction est réalisée dans un solvant inerte que l'on peut choisir parmi le toluène, le tétrahydrofurane ou encore le dichlorométhane ou le chloroforme, et, éventuellement, en présence de préférence d'une base organique ou minérale dont le rôle est de catalyser la réaction lorsque le dérivé d'acide optiquement actif engagé est un ester, ou encore de neutraliser les sous produits acides formés par la réaction. Les catalyseurs sont favorablement choisis parmi le sodium et ses alcoolates comme le méthylate de sodium qui est préféré, ou encore parmi les alcoolates métalliques comme ceux d'aluminium. Les bases utilisées pour la neutralisation peuvent être minérales comme des hydroxydes de métaux alcalins ou alcalino-terreux ou bien leurs sels parmi lesquels on

préfère le carbonate de sodium, ou encore être organiques comme des amines telles que des trialkylamines, des N,N dialkylanilines, des amines aromatiques. Parmi ces amines on préfère la triéthylamine et la pyridine.

Les diastéréoisomères obtenus après réaction sont séparés et purifiés par des méthodes habituelles à l'homme de l'art comme la cristallisation fractionnée ou une méthode de séparation chromatographique, puis ils sont hydrolysés sous l'action d'une base forte telle qu'un hydroxyde alcalin et en milieu habituellement hydroalcoolique, afin d'obtenir les énantiomères d'amino alcools séparés et purifiés et plus particulièrement dans le cadre de l'invention, l'eutomère de configuration (S) de formule (I).

c) La résolution du composé racémique ou la purification d'un mélange enrichi en eutomère (I) par chromatographie liquide de haute performance est réalisée par séparation des énantiomères sur une colonne contenant comme phase stationnaire une glycoprotéine acide dite $\alpha_1$-AGP immobilisée sur une charge de silice poreuse dont les sphères ont un diamètre moyen de 5 µm - colonnes CHIRAL-AGP (R) - (Société Chrom Tech).

La phase mobile servant à l'élution sélective est un mélange de solution tampon de pH 6 (solution de $KH_2$ $PO_4$ 0,01M ajustée à pH 6 par HOK N) et d'un solvant polaire miscible à l'eau, choisi parmi les alcools aliphatiques comprenant jusqu'à trois atomes de carbone et l'acétonitrile. Ce dernier solvant et l'isopropanol sont préférés, ils sont mélangés avec la solution tampon à raison de 0,1 à 25 % (v/v) pour éluer les énantiomères de la phase stationnaire. L'élution est réalisée dans des conditions appropriées à la bonne séparation des isomères, à savoir une température comprise entre 10 et 40° C et un débit compatible avec la résistance du matériel utilisé mais pouvant aller jusqu'à provoquer une pression de l'ordre de $10^7$ Pa.

Toutefois, les méthodes préférées de préparation des énantiomères de configuration (S) (I) sont celles mettant en oeuvre des énantiomères précurseurs de configuration (S) selon la règle établie par Cahn-Ingold-Prelog.

Le procédé préféré consiste à faire réagir un halogénure d'allyle sur un amino alcool précurseur de configuration (S) de formule :

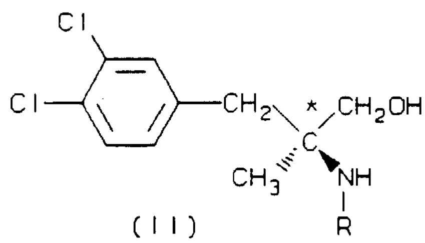

(II)

Un procédé alternatif consiste à réduire par un hydrure complexe dérivé du bore ou de l'aluminium la fonction carbonylée d'un amino-ester précurseur, de configuration (S), de formule

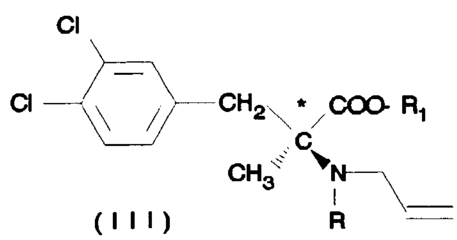

(III)

dans laquelle R est l'hydrogène ou méthyle et $R_1$ est l'hydrogène ou un radical alkyle inférieur comme méthyle ou éthyle.

En ce qui concerne la configuration (S) des composés de l'invention et des précurseurs (II) et (III), celle-ci a été attribuée par analogie à celle d'un précurseur qui leur est commun, le (S)-(-)-(dichloro-3,4-phényl)-3-méthyl-2-alaninate de méthyle de formule (IV)

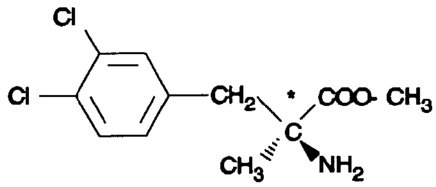

( I V)

dont la synthèse énantiosélective a été réalisée selon le procédé décrit par U.Schöllkopf (Synthesis, p. 969, 1981) et qui consiste à alkyler le (2R,5SR)-(-)-dihydro-2,5-dimethoxy-3,6-isopropyl-2-méthyl-5-pyrazine (produit Merck - réf. 818314) par le chlorure de 3,4 dichloro-benzyle, puis à réaliser une hydrolyse acide du (2R,5S)-(3,4 dichlorobenzyl)-5-dihydro-2,5-diméthoxy-3,6-isopropyl-2-méthyl-5-pyrazine pour obtenir l'ester méthylique de formule (IV) qui, par N-alkylation avec un halogénure d'allyle conduit au précurseur (III) dans lequel R est l'hydrogène, ou par réduction par un hydrure organométallique conduit au précurseur (II) dans lequel R est l'hydrogène ou méthyle, pour obtenir un eutomère (I).

Le procédé de préparation des composés (I) particulièrement préféré consiste :

- à faire réagir un halogénure d'allyle avec un amino alcool (II) dans lequel R est l'hydrogène pour préparer un eutomère (I) dans lequel R représente l'hydrogène,
- puis à réaliser une méthylation de cet eutomère (I) par le formaldéhyde et l'acide formique pour obtenir l'eutomère (I) dans lequel R représente un radical méthyle.

La mise en oeuvre de ce procédé consiste :

- à faire réagir le (S)-(-)-amino-2-(dichloro-3,4-benzyl)-2-propanol-1, composé décrit à l'exemple 1C du brevet européen N° 0 237 366, avec un halogénure d'allyle tel que le chlorure, l'iodure ou, de préférence, le bromure d'allyle, dans un solvant inerte tel que le toluène ou l'acétonitrile. Optionnellement, il est ajouté au milieu réactionnel un agent basique, minéral comme le carbonate de sodium, ou bien organique, comme la triéthylamine, pour favoriser la réaction. Pratiquement pour une mole d'amino alcool engagé, on utilise de 0,5 à 1,5 mole d'halogénure d'allyle, la réaction étant réalisée en solution dans 2 à 3 litres du solvant choisi. Selon le solvant et l'halogénure utilisé on obtient un résultat satisfaisant après une durée de réaction comprise entre 1 à 24 heures pour des conditions de température comprises entre 20 et 110° C. Les conditions préférées, en utilisant le bromure d'allyle, sont de 2 à 5 heures pour une température réactionnelle comprise entre 40 et 100° C, l'amino alcool (I) obtenu étant isolé et purifié par des méthodes habituelles notamment par cristallisation fractionnée, puis à N-méthyler ce composé (I) obtenu tel que décrit ci-dessus.

A cet effet, on réalise une réaction de méthylation réductrice avec une solution aqueuse de formaldéhyde et l'acide formique selon le procédé de Eschweiler-Clarke.

Pour une mole d'amino alcool (I) à N-méthyler, on met en oeuvre une quantité de solution aqueuse correspondant à 1,2 à 3,5 moles de formaldéhyde pur et de 2 à 5 moles d'acide formique pur.

Pratiquement on ajoute d'abord le formaldéhyde au produit puis, après réaction exothermique, l'addition de l'acide formique est effectuée et la réaction de réduction est réalisée par chauffage du mélange durant 1 à 2 heures à 90-100° C. Après traitements, l'eutomère (I) dans lequel R est méthyle est purifié par des méthodes conventionnelles, notamment chromatographiques.

En ce qui concerne la préparation du (S)-(-)amino-2-(dichloro-3,4-benzyl)-2-propanol-1, l'état de la technique est enseigné au brevet français N° 2 378 746 exemple 2-d, pour la synthèse de la l-(dichloro-3,4-phényl)-3-méthyl-2-alanine levogyre en 5 étapes à partir de l'isocyano-2-propionate de méthyle et du bromure de dichloro-3,4-benzyle, et par ailleurs, au brevet européen N° 0 237 366 exemple 1-C, où il est décrit la réduction de cet acide par le complexe borane-sulfure de diméthyle.

Avantageusement par rapport à cette préparation qui comprend au total six étapes, le procédé préféré qui est plus rapide et plus économique, permet de préparer le (S)-(-)-amino-2-(dichloro-3,4-benzyl)-2-propanol-1 en quatre étapes de synthèse à partir de la dichloro-3,4-phénylacétone. Essentiellement le procédé consiste à faire réagir par la réaction de Bucherer-Berg la cétone avec du cyanure de potassium et du carbonate d'ammonium pour obtenir la (+/-) (dichloro-3,4-benzyl)-5-méthyl-5-hydantoïne que l'on soumet en milieu hydro-acétonique alcalin à une salification stéréospécifique par la (R)-(+)-$\alpha$-méthylbenzylamine afin d'en obtenir le sel d'addition avec l'énantiomère (S) - (-) de l'hydantoïne, qui, insoluble est filtré et à partir duquel par traitement en solution acide on obtient la (S)-(-)-(dichloro-3,4-benzyl)-5-méthyl-5-hydantoïne dans un état de pureté optique supérieur à 98 %.

L'énantiomère (S) d'hydantoïne est hydrolysé de façon conventionnelle, en milieu aqueux alcalin, pour obtenir la (S)-(-)-(dichloro-3,4-benzyl)-2-alanine que l'on réduit tel que décrit à l'exemple 1 du brevet européen N° 0 237 366 par le complexe borane-sulfure de diméthyle pour obtenir le (S)-(-)-amino-2-(dichloro-3,4-benzyl)-2-propanol-1.

Ces procédés de préparation préférés sont illustrés par la préparation et les exemples décrits ci-dessous.

**Préparation** : (S)-(-)-amino-2-(dichloro-3,4-benzyl)-2-propanol-1 [(II) ; R= H]

- **Stade 1** : (+/-)-(dichloro-3,4-benzyl)-5-méthyl-5-hydantoïne Dans un réacteur, on introduit 160,0 g (0,79 mole) de 3,4-dichlorophénylacétone dans 700 ml d'éthanol et on chauffe le mélange à 40° C pour obtenir la dissolution. On ajoute ensuite 53,0 g (0,81 mole) de cyanure de potassium, 149,0g de sesquicarbonate d'ammonium et 700 ml d'eau.

  Le mélange est chauffé sous agitation à une température comprise entre 65 et 70°C durant 15 heures. La suspension est refroidie sous agitation à 10-15°C et abandonnée 16 heures à cette température. L'insoluble est filtré, lavé à l'eau puis à l'éther di-isopropylique avant d'être séché sous vide à 50°C jusqu'à poids constant.

  Poids obtenu 155,0 g    Rdt 72 %    F = 240°C

- **Stade 2** : (S)-(-)-(dichloro-3,4-benzyl)-5-méthyl-5-hydantoïne Dans un réacteur équipé en position de reflux, on mélange 100 ml d'eau déminéralisée avec 150ml d'acétone puis on ajoute 3,7 g (0,091 mole) d'hydroxyde de sodium en pastilles. On ajoute ensuite successivement 50,0 g (0,183 mole) de l'hydantoïne racémique obtenue au stade précédent et 22,2 g (0,183 mole) de (R)(+)$\alpha$-méthylbenzylamine.

  Sous agitation le mélange est porté au reflux jusqu'à dissolution des réactifs. On introduit alors 100 ml d'eau en chauffant le mélange progressivement jusqu'à 70° C, puis on refroidit lentement la solution sous agitation. Un début de cristallisation apparaît vers 60°C et le mélange est alors maintenu 1 h 30 à 50°C pour parfaire la cristallisation, puis refroidi à 20°C environ durant 30 minutes.

  Le précipité cristallin est filtré, lavé par 2 fois 100 ml d'un mélange acétone/eau 2-1 (vol/vol).

  L'insoluble est, tel quel, mis en suspension dans 250 ml d'eau et le mélange est acidifié à pH 1 par addition progressive d'une solution d'acide chlorhydrique concentré (d=1,18). L'insoluble est filtré, lavé à l'eau. La pureté optique du produit déterminé par CLHP sur une fraction aliquote est de 98,6 %.

  Le produit humide est recristallisé au reflux dans 200 ml d'un mélange eau-acétone 1 - 1 (vol/vol).

  Après refroidissement pendant deux heures à 15° C l'insoluble est filtré, lavé par un mélange acétone-eau puis séché sous vide à 50°C.

  On obtient 18,79 g de S-(-)-(dichloro-3,4-benzyl)-5-méthyl-5-hydantoïne.

| | | |
|---|---|---|
| Rendement = 75 %<br>F = 276 ° C | Pureté optique = 100 %<br>$[\alpha]_D^{20}$ = - 28,4° | (CLHP)<br>(c=1, EtOH) |

- **Stade 3 :** (S)-(-)-(dichloro-3,4-benzyl)-2-alanine

  Dans un autoclave on dissout 4,7 g (115 mmole) de soude en pastilles dans 25 ml d'eau. On ajoute 7,0 g (25,6 mmole) de la (S) (-) -hydantoïne préparée au stade 2 précédent. Après fermeture hermétique de l'appareil le mélange, est chauffé sous agitation durant 8 heures à 140-145°C sous une pression de 3 à 4 bars.

  Après refroidissement à 20-25°C la solution de couleur marron qui est obtenue est acidifiée sous agitation par une solution d'acide chlorhydrique concentré jusqu'à pH 5,8 - 6,2. Après refroidissement 30 minutes à 10° C l'insoluble est filtré, lavé à l'eau puis avec du toluène et séché sous vide à 80° C.

| | |
|---|---|
| Poids = 5,5 g<br>$[\alpha]_D^{20}$ = - 6,9° | Rdt = 86 %<br>(c = 1,5, $CH_3OH$ |

- **Stade 4:** (S)-(-)amino-2-(dichloro-3,4-benzyl)-2-propanol-1

  Dans un réacteur protégé de l'humidité et sous atmosphère d'azote, on introduit 62,5 ml de tétrahydrofurane (THF), puis 2,48 g (10 mmole) de (S)-(-)-(dichloro-3,4-benzyl)-2-alanine. Dans la suspension obtenue, en 30 minutes et à une température de 20° C, on ajoute goutte à goutte 3,80g (50 mmole) de complexe borane-diméthyl sulfure (BMS). L'agitation est maintenue 15 minutes à température ambiante, puis le mélange est chauffé 4 heures 30 au reflux. Après refroidissement à 5°C, 7,5 ml de méthanol sont introduits peu à peu sans dépasser 20°C, puis d'une façon identique 7,5 ml de solution d'hydroxyde de sodium N. La suspension obtenue est abandonnée une nuit, l'insoluble est filtré et éliminé. Le filtrat évaporé sous vide et sur bain-marie donne un résidu blanc qui est repris par 50 ml d'eau. Le mélange est acidifié jusqu'à pH 1 par addition d'acide chlorhydrique concentré (d=1,18).

La solution obtenue est extraite par deux fois 15 ml d'éther. Les phases éthérées sont écartées et la phase acide est alcalinisée à froid jusqu'à pH 12 par addition d'une solution d'hydroxyde de sodium concentrée (d = 1,38) puis saturée en chlorure de sodium.

Le mélange alcalin est extrait par 3 fois 20 ml d'éther, les phases éthérées réunies sont lavées par une solution saturée en chlorure de sodium, puis séchées.

Après évaporation de l'éther, l'amino alcool est obtenu sous forme d'un résidu solide, blanc et amorphe.

| | | |
|---|---|---|
| Poids 2,10 g<br>$[\alpha]^{20}_D$ = - 1,8° | F = 86°C<br>(c= 5 ; $CH_3OH$ | Rdt = 90 % |

**Exemple 1 -** (S)-(-)-N-allylamino-2-(dichloro-3,4-benzyl)-2-propanol-1 [(I) ; R = H]

Dans un réacteur protégé de l'humidité on dissout 35,0g (0,15 mole) de (S)-(-)amino-2-(dichloro-3,4-benzyl)-2-propanol-1 $[\alpha]_D^{20}$ = -1,8° (c = 5, méthanol) dans 350 ml d'acétonitrile anhydre.

A la solution, on ajoute sous agitation à 20-25° C, et en 5 minutes environ, 12,9 ml (18,08 g - 0,15 mole) de bromure d'allyle. La solution est maintenue sous agitation une heure à la même température, puis chauffée progressivement à 50-60° C. Cette température est maintenue deux heures et demi, période au cours de laquelle un précipité apparaît. La suspension est ensuite refroidie par un bain d'eau glacée à 5° C. Le précipité est alors filtré, repris par 800ml d'eau, alcalinisé jusqu'à pH 11 par une solution 10 N d'hydroxyde de sodium et extrait par 3 fois 250 ml d'éther diéthylique.

Les phases éthérées réunies sont extraites par une solution saturée en NaCl puis déshydratées sur $Na_2SO_4$. L'éther est évaporé sous vide et sur bain marie. Le résidu solide brut est repris pour dissolution dans 500 ml d'hexanes au reflux. Après refroidissement le précipité blanc cristallin de (S)-(-)-N-allylamino-2-(dichloro-3,4-benzyl)-2-propanol-1 purifié, est filtré puis porté en étuve à 40°C sous vide jusqu'à poids constant.

| | | |
|---|---|---|
| Poids : 25,4 g<br>$[\alpha]_D^{20}$ = - 12° | F = 87-88°C<br>(c = 0,5 , HCl N) | Rdt 62 % |

note : déviation observée non significative pour :
(C = 5, $C_2H_5OH$) et (c = 1, $C_2H_5OH$/HCl N)

- $^1H$ - RMN ($CDCl_3$ TMS) : δ (ppm) 1,00 (s, 3H) ; 1,00 - 2,90 (m, 2H éch. $D_20$) ; 2,65 (s, 2H) ; 3,10 - 3,40 (m, 4H) ; 5,00 - 5,35 (m, 2H) ; 5,65 - 6,15 (m, 1H) ; 6,90 - 7,45 (m, 3H).

**Exemple 2 -** (S)-(+)-N-allylméthylamino-2-(dichloro-3,4-benzyl)-2-propanol-1 [(I) ; R = $CH_3$]

Dans un réacteur muni d'une agitation énergique on mélange 13,5 g (49 mmole) du (S)-N-allylamino propanol préparé à l'exemple 1 précédent, avec 13,5 ml de solution de formaldéhyde à 37 % p/v (soit 5,0 g - 133 mmole).

Le mélange est tiédi vers 40-50°C pour favoriser l'homogénéisation sous agitation, puis refroidi à 15°C. On ajoute alors goutte à goutte 9,3 ml d'acide formique à 100 % (d = 1,22 soit 11,35 g - 247 mmole). La solution limpide obtenue est maintenue deux heures et demi sous agitation sur bain marie bouillant. Après refroidissement, on ajoute 200 ml d'eau, puis acidifie à pH 1 par addition d'acide chlorhydrique concentré et extrait par 3 fois 100 ml d'éther diéthylique.

Les phases éthérées sont écartées, la phase acide est alcalinisée jusqu'à pH 11 sans dépasser 25°C par une solution 10 N d'hydroxyde de sodium, puis le mélange est extrait par 3 fois 150 ml d'éther.

Les phases éthérées réunies sont lavées par une solution de NaCl puis séchées sur $Na_2SO_4$. L'éther est évaporé, le résidu brut de 14,0 g est purifié selon la technique chromatographique dite de "Chromatoflash" sur une colonne garnie de silice.

L'élution par le mélange dichlorométhane-méthanol 95-5 (v/v) permet d'obtenir le (S)-(+)-N-allylméthylamino-2-(dichloro-3,4-benzyl)-2-propanol-1 purifié sous une forme huileuse qui cristallise lentement en un produit de bas point de fusion.

Poids = 11,5 g Rdt = 81 %
$[\alpha]_D^{20}$ = + 13° (c = 1, HCl N)

- $^1H$ - RMN ($CDCl_3$ - TMS) : δ (ppm) 0,95 (s, 3H) ; 2,30 (s;3H) 2,70 (s, 2H) ; 2,95 (s; 1H éch.$D_20$) ; 3,05 - 3,45 (m, 4 H) ; 5,00 - 5,35 (m, 2H) ; 5,60 - 6,05 (m, 1 H) ; 6,95 - 7,40 (m, 3H)
- CLHP - Détermination de la pureté optique sur colonne "Chiral -AGP (R) - fournisseur Chromtech" en éluant par un mélange $KH_2$ $PO_4$ 0,01M ajusté à pH6 - Isopropanol - (v/v) résultat : pureté % > 98

- Chlorhydrate : l'eutomère (I) obtenu précédemment est dissout dans 150 ml de dichlorométhane. En maintenant une température inférieure à 25°C, on ajoute à la solution un excès d'éther chlorhydrique puis abandonne sous agitation 1 heure en protégeant de l'humidité.

Les solvants sont évaporés sous vide et sur bain-marie. Le résidu solide est dissout dans la quantité minimale de méthanol à 20-25° C et le chlorhydrate purifié est obtenu par précipitation après addition d'éther diéthylique anhydre.

F = 171-172°C

- Analyse élémentaire ($C_{14}\, H_{20}\, Cl_3\, NO$ = 324,69)

| | | | | | |
|---|---|---|---|---|---|
| Calculé | C 51,79 | H 6,21 | Cl 32,76 | N 4,31 | O 4,93 |
| Trouvé | C 51,71 | H 6,30 | Cl 32,72 | N 4,20 | O 5,22 |

- IR (KBr) : 3250, 2600, 1470, 1400, 1060, 1030, 950, 830 $cm^{-1}$

Les antipodes distomères des composés présentés aux exemples 1 et 2 sont respectivement :

- le (R) - (+) -N-allylamino-2- (dichloro-3,4-benzyl) ) - 2 -propanol - 1
  F =89°C $[\alpha]_D^{20}$ = + 11° (c = 0,5 , HCl N)
- le (R) - (-) -N-allylméthylamino-2- (dichloro-3,4-benzyl) -2-propanol-1

F < 50°C $[\alpha]_D^{20}$ = - 14° (c = 1, HCl N)

Ils ont été préparés selon le mode opératoire décrit aux exemples prédécents à partir du (R)-(-)-amino-2-(dichloro-3,4-benzyl)-2-propanol-1 décrit à l'exemple 1 B du brevet européen N° 0 237 366.

La faible toxicité des produits de formule (I) et les propriétés pharmacologiques inattendues qui ont été découvertes justifient de leur utilité à la préparation de médicaments destinés aux traitements préventif ou curatif des manifestations allergiques et notamment de celles provoquées par une libération d'histamine.

La toxicité aigüe des composés (I) a été étudiée par voie orale chez la souris mâle.

A cet effet, les produits ont été administrés en solution aqueuse à raison de 2ml pour 100 g de poids corporel. Les animaux ont été observés durant les trois heures qui suivent l'administration puis quotidiennement durant 14 jours, après quoi ils ont été sacrifiés et autopsiés.

Les DL50 (doses léthales provoquant la mort de 50 % des animaux) sont aux environs de 1.000 mg/kg ce qui peut être apprécié comme une toxicité relativement faible des produits.

Les propriétés antiallergiques des eutomères (I) ont été mises en évidence par leur aptitude à inhiber, chez le cobaye sensibilisé à l'ovalbumine, un effet bronchoconstricteur anaphylactique provoqué par un aérosol contenant ce même allergène.

La technique pratiquée consiste à sensibiliser au préalable des cobayes de 250 à 300 g par deux injections intrapéritonéales, pratiquées à 24 heures d'intervalle, de 0,5 ml d'une suspension stérile de sérum physiologique (NaCl 0,9 %) contenant 20 µg d'ovalbumine (Grade V ; Sigma) et 100 mg d'hydroxyde d'aluminium $Al(OH)_3$.

En même temps des animaux témoins non sensibilisés sont traités par deux injections de suspension ne contenant pas l'ovalbumine.

L'expérience proprement dite s'effectue après une période nécessaire à la sensibilisation des animaux qui est comprise entre 14 et 21 jours. Elle est généralement atteinte après 18 jours et consiste d'abord à administrer par voie orale à des lots de 5 animaux des quantités croissantes du produit à l'essai en solution dans l'eau et ce à raison de 0,5 à 1,0 ml par animal correspondant à des doses de 1 à 10 µmol/kg de produit.

Les animaux sont ensuite anesthésiés 50 minutes après cette administration par une injection intrapéritonéale d'une solution d'uréthane. Ils subissent alors une préparation chirurgicale qui consiste à :

- placer une canule dans la carotide gauche afin de pouvoir déterminer en continu la pression sanguine et la fréquence cardiaque,
- placer une canule dans la veine jugulaire droite pour l'administration intraveineuse de solutions,
- après une trachéotomie cervicale supérieure, insérer une canule de polystyrène (diamètre intérieur 2,65 mm, longueur 9 à 11 mm) afin de ventiler l'animal (pompe Harvard 50-1728 ; Kent-Grande-Bretagne) à une fréquence

de 60 insufflations par minute et à raison d'un volume de 1,2 ml/kg chaque.

Après cette intervention et stabilisations de la pression intratrachéale et de la pression sanguine un aérosol d'ovalbumine est administré au moyen d'un nébulisateur à ultra sons (appareil Pulmosonic ; modèle 2511). L'appareil délivre par la ventilation trachéale en 30 secondes 30 insufflations sous un volume total de 30µl d'une suspension d'ovalbumine à 5 mg/ml. Après 60 minutes, la pression intratrachéale mesurée en mm de Hg est déterminée pour chaque animal des lots d'essai et ceux du lot témoin. Ces mesures permettent de déterminer l'importance de l'inhibition de la bronchoconstriction provoquée par le produit à l'essai. Les résultats sont exprimés par la $DE_{50}$ du produit qui est la dose efficace, exprimée en micromoles par kg (µmol/kg) capable d'inhiber 50 % de la bronchoconstriction provoquée par l'ovalbumine chez l'animal sensibilisé dans les conditions de l'essai.

Déterminée dans ces conditions la $DE_{50}$ inhibitrice du chlorhydrate de l'eutomère (I) de l'exemple 2 est de 1,84 µmol/kg.

Par ailleurs, les propriétés antihistamiques ont été étudiées chez le cobaye par le test de bronchoconstriction pratiqué d'après la méthode de H. Konzett et R. Rossler (Arch. Exp. Path. Pharmak. - Naunym Schmiedeberg -195, 71-74, 1940).

Les produits de l'invention ont été étudiés dans ce test après administration en solution ou sous forme de suspension , par les voies intraveineuse et intraduodénale et aussi sous forme de poudre par voie intranasale.

Quelle que soit la voie d'administration, la préparation des animaux engagés dans l'essai consiste d'abord à anesthésier des cobayes mâles d'un poids de 350 à 400 g par injection intrapéritonéale (i.p.) d'une solution stérile et isotonique à 25 % (p/v) de carbamate d'éthyle à raison de 6,0 ml/kg, puis à appareiller les animaux par une canule trachéale pour permettre la mesure de la pression pulmonaire, une canule dans la veine jugulaire droite pour l'administration intraveineuse (i.v.) de solution et également une canule dans l'artère carotide gauche pour l'observation qualitative de la pression sanguine.

Les animaux sont ensuite connectés à une pompe (Harvard, réf. 50-1718) afin de maintenir une ventilation artificielle à une fréquence de 60 insufflations par minute ; la pression intratrachéale est enregistrée par un ensemble comprenant un transducteur, un amplificateur et un enregistreur (Gould, réf. respectives : PLOEZ, 13-4615-50 et 8188-G4400-06).

Ainsi appareillés les animaux sont laissés au repos durant 10 minutes avant le début de l'expérimentation proprement dite qui est réalisée de façon différente selon la voie d'administration envisagée.

a) administration par voie i.v.

Sous forme de base, les produits à l'essai sont dissous dans une solution isotonique (NaCl 0,9 %) contenant 10 % (v/v) de Transcutol (R). Les dilutions sont effectuées par ajout de soluté isotonique. Sous forme de sels, notamment de chlorhydrate, les solutions sont préparées dans le soluté isotonique.

Le traitement des animaux consiste en une série d'administrations de solution de dichlorhydrate d'histamine à raison de 100 µmol/kg avant et après le traitement par la solution de produit à l'essai, ce qui est réalisé selon la chronologie suivante :

| | |
|---|---|
| t = - 20 minutes | - histamine (100µmol/kg) |
| t = - 10 minutes | - id. contrôle |
| t = 0 | - injection du produit à l'essai |
| t = + 10 minutes | - histamine (100 µmol/kg) |
| t = + 20 minutes | - id. |
| t = + 30 minutes | - id. |
| t = + 40 minutes | - id. |
| t = + 50 minutes | - id. |
| t = + 60 minutes | - id. |

Les résultats des essais sont présentés au tableau 1.

b) administration par voie i.d.

Sous forme de base, les produits à l'essai sont mis en suspension dans une solution à 1 % p/v de carboxy-méthylcellulose (CMC), et, sous forme de sels, ils sont dissous dans un soluté isotonique avant d'être administrés par l'intermédiaire d'une canule dont l'extrémité est placée au niveau duodénal de l'animal.

La chronologie des administrations succesives d'histamine par voie i.v. et celle de la solution d'essai par voie i.d. sont identiques à celle décrite précédemment en a).

Les résultats de ces essais sont présentés au tableau 2.

c) administration de poudre par voie intranasale (In.p).

Le produit à l'essai est administré par une insufflation nasale d'une poudre constituée par 20 mg de lactose chargé avec la quantité appropriée du produit à l'essai. Avant cette insufflation, la ventilation artificielle de l'animal est momentanément stoppée pour faciliter la pénétration pulmonaire du composé. La chronologie de l'administration par voie i.v. de l'histamine et celle par voie In.P. du produit à l'essai est la suivante :

| | |
|---|---|
| t = - 15 minutes | - histamine (100 µmol/kg) |
| t = - 5 minutes | - id. contrôle |
| t = 0 | - insufflation produit à l'essai |
| t = 5 minutes | - histamine (100 µmol/kg) |

Les résultats de ces essais sont présentés au tableau 3.

- Calcul et expression des résultats :
Pour chaque voie d'administration et pour chaque temps d'administration d'histamine l'activité des produits est exprimée par le pourcentage de variation d'amplitude de bronchoconstriction calculé par rapport à celle provoquée par l'administration "contrôle" d'histamine qui précède l'administration du produit à l'essai. Le % de variation est calculé selon la formule :

$$\% \text{ variation} = 100 - \left(\frac{Bc\,(tx) - Bo\,(tx)}{Bc\,(to) - Bo\,(to)} \times 100\right)$$

dans laquelle :

Bc(tx) représente en mm l'amplitude de la bronchoconstriction au temps x,
Bo(tx) l'amplitude en mm de la respiration basale au temps x,
Bc(to) et Bo(to) les amplitudes correspondantes en mm lors de l'administration "contrôle" de l'histamine qui précède celle du produit à l'essai.

L'activité inhibitrice des produits à l'essai sur la bronchoconstriction provoquée par l'histamine est exprimée aux différents temps des études par leur $DE_{50}$ qui est la dose efficace de produit exprimé en µmol/kg capable d'inhiber au temps considéré 50 % de la bronchoconstriction provoquée par l'histamine.

A défaut de cette expression, les résultats sont exprimés en % de variation au temps considéré et pour une concentration donnée du produit à l'essai. Dans ces conditions, un pourcentage positif correspond à un effet potentialisateur de la bronchoconstriction, et, inversement, un pourcentage négatif à un effet inhibiteur.

Les analyses statistiques des résultats ont été réalisées par le test *t* de Student. Une valeur de $p < 0,05$ est considérée significative.

Les composés décrits aux exemples 1 et 2 ont été engagés dans ces essais. Egalement, pour produit de comparaison à l'eutomère de l'exemple 1, il a été engagé le composé racémique (R,S) qui lui correspond et qui est décrit et préparé à l'exemple 7 du brevet européen N° 0 237 366. En ce qui concerne l'eutomère de l'exemple 2 et son chlorhydrate il a été engagé comparativement dans ces essais le chlorhydrate du composé racémique (R,S) et qui est décrit à l'exemple 13 du brevet européen N° 0 237 366 et également l'antipode distomère de configuration (R) correspondant préparé tel que décrit précédemment.

Le chlorhydrate de Fenspiride (DCI), connu comme antagoniste d'allergènes actifs sur les voies respiratoires, et le Cromakalim (DCI) qui est un composé réputé bronchodilatateur, ont été engagés également dans ces essais au titre de composés de référence.

- Essais par voie i.v. (Résultats : $DE_{50}$ µmol/kg)

| COMPOSE | Config. | t=10' | t=20' | t=30' | t=40' | t=50' | t=60' |
|---|---|---|---|---|---|---|---|
| - Composé de l'exemple 1 | (S) | 4,89 | 2,88 | 2,54 | 2,58 | 3,17 | 3,55 |
| Racémique comparatif | (R,S) | 6,65 | 4,10 | 4,38 | 3,39 | 3,02 | 3,52 |
| - Composé de l'exemple 2 | | | | | | | |
| - base | (S) | 1,27 | 0,93 | 0,74 | 0,96 | 1,01 | 0,76 |
| - chlorhydrate | (S) | 1,68 | 0,70 | 0,58 | 0,57 | 0,51 | 0,53 |
| Racémique comparatif, HCl | (R,S) | 3,72 | 2,11 | 1,88 | 2,07 | 2,34 | 2,22 |
| Distomère comparatif * | (R) | + 6,3 | + 18,4 | + 28,2 | + 35,4 | + 36,7 | + 41,1 |
| Référence : | | | | | | | |
| Fenspiride (DCI), HCl | - | 4,25 | 4,99 | 6,26 | 8,90 | 8,01 | 11,03 |

* % effets potentialisateurs de la bronchoconstriction statistiquement non significatifs pour 3,5 µmol/kg.

Tableau 1 : Effet inhibiteur, par voie i.v., sur la bronchoconstriction provoquée par 100 µmol/kg d'histamine

- essai par voie i.d. (Résultats : $DE_{50}$ µmol/kg)

| Composé | Config. | t=10' | t=20' | t=30' | t=40' | t=50' | t=60' |
|---|---|---|---|---|---|---|---|
| - Composé de l'exemple 1 | (S) | - | 1,00 | 0,78 | 0,69 | 1,28 | 2,17 |
| - Composé de l'exemple 2 | | | | | | | |
| - base | (S) | - | 1,45 | 0,59 | 0,65 | 0,63 | 0,62 |
| - chlorhydrate | (S) | 1,59 | 0,42 | 0,33 | 0,48 | 0,51 | 0,56 |
| Distomère comparatif * | (R) | + 5,8 | + 14,6 | + 32,4 | + 44,5 | + 35,6 | + 30,0 |

* % effets potentialisateurs de la bronchoconstriction statistiquement non significatifs pour 3,5µmol/kg

Tableau 2 : Effet inhibiteur, par voie i.d., sur la bronchoconstriction provoquée par 100 µmol/kg d'histamine

- essai par voie In.P (Résultats $DE_{50}$ µmol/kg)

Tableau 3 :

| Effet inhibiteur, par voie In.P. (intranasale-poudre), sur la bronchoconstriction provoquée par 100nmol/kg d'histamine | | |
|---|---|---|
| Composé | Config. | $DE_{50}$ à t = 5 mn |
| - Composé de l'exemple 2 | | |
| - base | (S) | 1,28 |
| - chlorhydrate | (S) | 1,77 |
| Cromakalim (DCI) | | 1,88 |

Les résultats de ces essais sont probants de l'activité inhibitrice des composés à l'essai sur la bronchoconstriction provoquée par l'ovalbumine et l'histamine chez le cobaye. Egalement, ils mettent en évidence la spécificité de l'activité antihistaminique des composés des exemples 1 et 2 de configuration (S) qui sont appliqués dans l'invention. Ainsi, comparativement à leurs analogues racémiques (R,S) ces composés se montrent pratiquement au moins deux fois plus actifs.

Par ailleurs, par voie i.v. ou In. les composés (I) à l'essai montrent une activité égale sinon supérieure aux composés de référence auxquels ils sont comparés.

D'une façon plus surprenante, on constate qu'en ce qui concerne le produit de l'exemple 2, aussi bien par voie i. v. que par voie i.d., le distomère correspondant de configuration (R) montre, de façon perceptible bien que non statistiquement significative, un effet potentialisateur de l'activité de l'histamine.

Ces propriétés antiallergiques et notamment antihistaminiques dans le test de bronchoconstriction apparaissent non évidentes au regard de l'enseignement de l'état de la technique et remarquables au regard de la dissociation d'activité constatée entre les eutomères de formule (I) et leurs antipodes.

Elles justifient l'objet de l'invention qui concerne au titre d'application nouvelle les énantiomères (I) de configuration S qui sont :
- le(S)-(-) -N-allylamino-2- (dichloro-3,4-benzyl)-2-propanol-1 - le (S) - (+) -N-allylméthylamino-2- (dichloro-3,4-benzyl) -2-propanol-1,
et leurs sels, pour leur utilisation à la préparation de médicaments destinés aux traitements préventifs ou curatifs des états allergiques et notamment de ceux impliquant une libération d'histamine.

Sous la forme de compositions pharmaceutiques appropriées, les composés par leur activité antiallergique sont utiles aux traitements des états asthmatiques et notamment à inhiber les états de bronchoconstriction ou de bronchospasme de l'asthme allergique ou de ceux résultant de bronchites aigües ou chroniques. L'activité antihistaminique des composés justifie leur application aux symptomatologies provoquées par la libération d'histamine comme par exemple dans les allergies des muqueuses nasales et conjonctives, les rhinites allergiques, certaines formes d'oedèmes, de dermatoses, de purits ou d'eczéma.

La faible toxicité des produits autorise pour obtenir les effets attendus des posologies journalières pouvant atteindre jusqu'à 1000 mg par voie orale. Habituellement, elles sont toutefois de 50 à 500 mg par jour, par voie orale, réparties si nécessaire en plusieurs prises.

Les médicaments obtenus à partir des produits (I) et leurs sels pharmaceutiquement acceptables sont administrés par les voies adaptées à la nature et à l'importance de l'affection à traiter.

Ces médicaments sont par exemple des comprimés, dragées, capsules, poudres, des suppositoires, des gels, des suspensions ou encore des solutions injectables ou buvables.

Ils sont préparés par des méthodes courantes à l'homme de l'art. Ils comprennent de 1 à 50 % en poids de principe actif constitué par un composé (I) ou un de ses sels et de 99 à 50 % en poids d'un véhicule pharmaceutiquement approprié et compatible avec les principes actifs et la forme physique de la composition envisagée.

A titre d'exemples non limitatifs la préparation de comprimés et de solutés isotoniques injectables avec les composés (I) est présentée.

**Comprimés**

- Formule

| | |
|---|---|
| Substance active (I) selon l'exemple 2 | 5 à 75 mg |
| Polyvinylpyrrolidone | 2 mg |

(suite)

| | |
|---|---|
| Carboxyméthylamidon | 8 mg |
| Stéarate de magnésium | 3 mg |
| Lactose | 60 à 76 mg |
| Cellulose monocristalline pour un comprimé de 200 mg. | 122 à 76 mg |

- Fabrication

  Dissoudre la polyvinylpyrrolidone à raison de 0,1 à 1,0 % en poids dans l'eau, un alcool de bas poids moléculaire comme l'éthanol ou un mélange hydroalcoolique.

  Par ailleurs mélanger intimement la substance active, le lactose, la moitié de la quantité de cellulose et du carboxyméthylamidon et humidifier ce mélange avec la solution obtenue précédemment.

  Granuler la pâte, sécher les granulés pour les calibrer sur tamis. Ajouter le reste des composants, mélanger intimement puis comprimer à raison de 200 mg par unité.

**Soluté isotonique injectable**

- Formule

| | |
|---|---|
| Substance active (I), chlorhydrate de l'exemple 2 | 10 mg |
| Chlorure de sodium | 9 mg |
| Eau distillée en quantité suffisante pour | 1,0 ml |

- Fabrication

  Le soluté est réparti en ampoules qui sont après scellement stérilisées par les moyens thermiques habituels ; le soluté peut également être stérilisé par filtration, réparti en ampoules qui sont ensuite scellées, ces opérations étant effectuées en atmosphère stérile.

**Revendications**

1. Utilisation d'énantiomères de configuration absolue (S) selon la règle de Cahn-Ingold-Prelog, de formule (I)

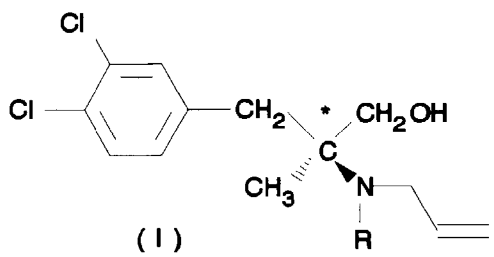

(I)

dans laquelle,
R est l'hydrogène ou un radical méthyle, et leurs sels d'addition avec les acides non toxiques pour l'obtention de médicaments destinés au traitement d'états allergiques.

2. Utilisation selon la revendication 1 du (S)-(+)-N-allylméthylamino-2- (dichloro-3,4-benzyl) - 2 -propanol - et ses sels d'addition avec les acides non toxiques.

3. Utilisation selon les revendications 1 ou 2 caractérisée en ce que les médicaments obtenus soient destinés aux traitements d'allergies provoquées par l'histamine.

**Patentansprüche**

1. Verwendung von Enantiomeren in der nach der Cahn-Ingold-Prelog-Regel absoluten (S)-Konfiguration der Formel (I)

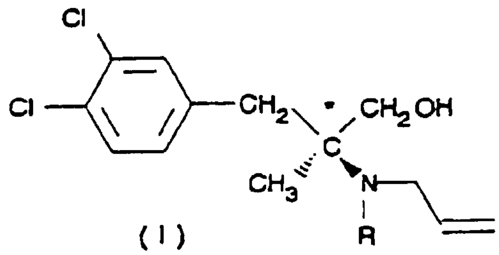

(I)

worin
R Wasserstoff oder einen Methylrest bedeutet, und deren Additionssalze mit ungiftigen Säuren zur Herstellung von Arzneimitteln, die zur Behandlung von Allergiezuständen bestimmt sind.

2. Verwendung nach Anspruch 1 von (S)-(+)-2-N-Allylmethylamino-2-(dichlor-3,4-benzyl)-1-propanol und dessen Additionssalze mit ungiftigen Säuren.

3. Verwendung nach Anspruch 1 oder 2, dadurch gekennzeichnet, daß die erhaltenen Arzneimittel zur Behandlung von durch Histamin hervorgerufenen Allergien bestimmt sind.

**Claims**

1. Use of enantiomers of (S) absolute configuration according to the Cahn-Ingold-Prelog rule, of formula (I)

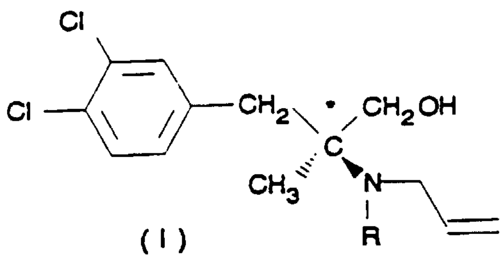

(I)

in which,
R is hydrogen or a methyl radical, and the addition salts thereof with non-toxic acids, for the production of medicinal products intended for the treatment of allergic states.

2. Use, according to Claim 1, of (S)-(+)-2-N-allylmethylamino-2- (3,4-dichlorobenzyl)-1-propanol and the addition salts thereof with non-toxic acids.

3. Use according to Claims 1 and 2, characterized in that the medicinal products obtained are intended for the treatment of histamine-induced allergies.